# EUROPEAN PATENT APPLICATION

(11) **EP 4 155 290 A1**
(43) Date of publication of application: **29.03.2023**
(21) Application number: 21809182.5
(22) Date of filing: 20.05.2021
(51) Int. Cl.: C07C 67/32, C07C 67/03, C07C 69/14, C07C 69/68

(54) **METHOD FOR PRODUCING ESTER COMPOUND**

(30) Priority: 21.05.2020 JP 2020088891
(71) Applicant: Kyushu University, National University Corporation, Nishi-ku Fukuoka-shi Fukuoka 819-0395 (JP); Showa Denko K.K., Tokyo 105-8518 (JP)
(72) Inventor: TOKUNAGA Makoto, Fukuoka-shi, Fukuoka 819-0395 (JP); MURAYAMA Haruno, Fukuoka-shi, Fukuoka 819-0395 (JP); YAMAMOTO Eiji, Fukuoka-shi, Fukuoka 819-0395 (JP); SHIM Jooyoung, Fukuoka-shi, Fukuoka 819-0395 (JP); MORI Haruki, Fukuoka-shi, Fukuoka 819-0395 (JP); SHIRAKURA Nao, Fukuoka-shi, Fukuoka 819-0395 (JP); TSUKAMOTO Shinya, Tokyo 105-8518 (JP); NISHIZAWA Shohei, Tokyo 105-8518 (JP); KITAGAWA Kazuhiro, Tokyo 105-8518 (JP); UCHIDA Hiroshi, Tokyo 105-8518 (JP)
(74) Representative: Strehl Schübel-Hopf & Partner
(86) International application number: PCT/JP2021/019162
(87) International publication number: WO 2021/235518

(57) **Abstract**

What is provided is a production method in which a vinyl acetate is reacted with a primary or secondary alcohol represented by Formula (1) and carbon monoxide to produce a first ester compound represented by Formula (2), and the first ester compound is reacted with an alcohol to produce a lactic acid ester represented by Formula (3) and an acetic acid ester represented by Formula (4).

R¹OH (1)

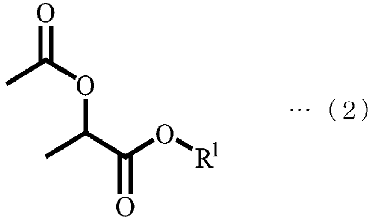

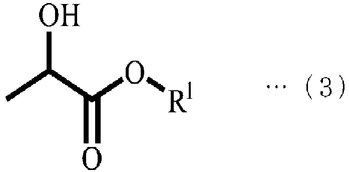

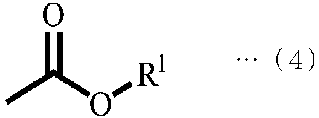

## Description

### TECHNICAL FIELD

The present invention relates to a method for producing an ester compound.

Priority is claimed on Japanese Patent Application No. 2020-088891, filed May 21, 2020, the content of which is incorporated herein by reference.

### BACKGROUND ART

An ester compound has been used as an intermediate raw material for solvents, fragrances, emulsifiers, moisturizers, pharmaceuticals, and the like. Recently, lactic acid ester, which is the ester compound, has attracted attention as a solvent for resists and paints, and as a cleaning agent.

Patent Document 1 discloses a method of carbonylating a vinyl acetate, that the vinyl acetate is reacted with carbon monoxide in the presence of a source of a hydroxyl group and a catalytic system in which (a) a metal of Group V111B or a compound thereof and (b) a specific bidentate phosphine are combined.

Patent Document 2 discloses a method of producing a propionic acid ester derivative, that a substituted vinyl compound is reacted with an alcohol compound and carbon monoxide in the presence of a compound of Periodic Table Group 8 metal, a phosphorus ligand, and a polymer sulfonic acid compound.

Patent Document 3 discloses a method of producing a propionic acid ester derivative, that an olefinically unsaturated compound is reacted with alcohols and carbon monoxide in the presence of at least one of a compound of Group 10 metal or a neutral co-catalyst.

Patent Document 4 discloses a method of producing 2-hydroxycarboxylic acid, which includes a step (a) of reacting an enol ester with carbon monoxide and a hydroxyl compound in the presence of a palladium catalyst and a solvent to obtain a carbonylated ester and a step (b) of hydrolyzing the carbonylated ester with an acid catalyst to obtain 2-hydroxycarboxylic acid. Patent Document 4 discloses vinyl acetate as the enol ester, dichlorobis(triphenylphosphine)palladium (II) (PdCl₂(PPh₃)₂) of the palladium catalyst, methanol as the hydroxyl compound, and lactic acid as the 2-hydroxycarboxylic acid.

Patent Document 5 discloses a method of producing 2-hydroxy acid, which includes a step (1) of reacting an enol acylate with carbon monoxide and a hydroxyl compound to produce an acyloxy ester and/or a hydroxy ester and a step (2) of hydrolyzing the product of (1) to produce 2-hydroxy acid.

Patent Document 6 discloses a method including a step of reacting a compound having an unsaturated covalent bond between two carbon atoms with carbon monoxide in the presence of a source of a hydroxyl group and a catalytic system. Patent Document 6 discloses vinyl acetate as the compound having an unsaturated covalent bond between two carbon atoms.

In addition, Non-Patent Document 1 discloses a method of reacting an olefin compound (enol ester) with an alcohol (methanol) and carbon monoxide using a palladium complex catalyst in the presence of a basic co-catalyst such as a pyridine derivative.

Non-Patent Document 2 discloses a methoxycarbonylation reaction of vinyl acetate using a palladium catalyst and a monodentate ligand such as triphenylphosphine.

### CITATION LIST

### Patent Documents

[Patent Document 1]
   Published Japanese Translation No. 2006-508162 of the PCT International Publication
[Patent Document 2]
   Japanese Unexamined Patent Application, First Publication No. 2005-247703
[Patent Document 3]
   Japanese Unexamined Patent Application, First Publication No. 2006-225282
[Patent Document 4]
   United States Patent Application, Publication No. 2005/0143600
[Patent Document 5]
   Japanese Unexamined Patent Application, First Publication No. S60-149544
[Patent Document 6]
   Japanese Unexamined Patent Application, First Publication No. 2015-110608
[Patent Document 7]
   Japanese Unexamined Patent Application, First Publication No. 2005-206468
[Patent Document 8]
   Japanese Unexamined Patent Application, First Publication No. H5-287282
[Patent Document 9]
   Japanese Unexamined Patent Application, First Publication No. 2001-192675

### Non-Patent Documents

[Non-Patent Document 1]
   Bulletin of the Chemical Society of Japan, 1996, vol. 69, pp. 1337 to 1345
[Non-Patent Document 2]
   ARKIVOC, 2012, (iii) pp. 66 to 75
[Non-Patent Document 3]
   NEDO Overseas Report No. 977 (2006.4.26), pp. 58 to 60
[Non-Patent Document 4]
   Chemical process corpus, Recycling of waste plastic - producing raw material by gasification; [Search on May 20, 2020] Internet <URL: http://www3.scej.org/education/gasifi.html>

### SUMMARY OF INVENTION

### Technical Problem

However, in the methods for producing an ester compound in the related art, a lactic acid ester and an acetic acid ester cannot be produced efficiently.

The present invention is made in consideration of the above-described circumstances, and an object of the present invention is to provide a method for producing an ester compound, in which a lactic acid ester and an acetic acid ester can be efficiently produced.

Another object of the present invention is to provide a method for producing an ester compound, in which an ester compound can be produced in a high yield.

### Solution to Problem

The present inventors have diligently studied in order to achieve the above-described objects.

As a result, the present inventors have found that a specific ester compound may be produced by reacting vinyl acetate with a specific alcohol and carbon monoxide and the specific ester compound may be reacted with an alcohol, and the present invention has been conceived.

That is, the present invention relates to the following matters.
[1] A method for producing an ester compound, comprising:
   a first step of reacting a vinyl acetate with a primary or secondary alcohol represented by General Formula (1) and carbon monoxide in a presence of a first catalyst to produce a first ester compound represented by General Formula (2); and
   a second step of reacting the first ester compound with an alcohol in a presence of a second catalyst to produce a lactic acid ester represented by General Formula (3) and an acetic acid ester represented by General Formula (4).
   R¹OH ··· (1)

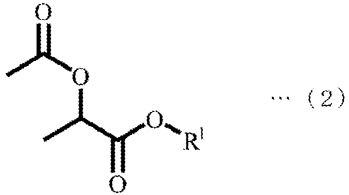

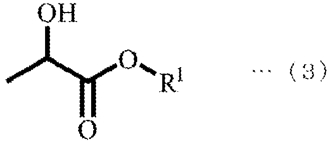

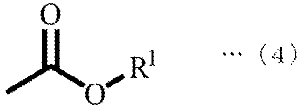

   (in Formulae (1) to (4), R¹ is a hydrocarbon group which has 1 to 10 carbon atoms and may have an alicyclic ring or an aromatic ring in part or in whole, R¹'s in Formula (1) and Formula (2) are the same, and R¹'s in Formula (3) and Formula (4) are the same)
[2] The method for producing an ester compound according to [1], further comprising, before the second step:
   an intermediate step of reducing an unreacted vinyl acetate included in a reaction solution after the first step to convert the unreacted vinyl acetate into an acetic acid ester.
[3] The method for producing an ester compound according to [2],
   wherein, in the intermediate step, an acetaldehyde included in the reaction solution after the first step is hydrogenated to be converted into ethanol, and
   in the second step, the first ester compound in the reaction solution after the intermediate step is reacted with the ethanol in the presence of the second catalyst.
[4] The method for producing an ester compound according to any one of [1] to [3],
   wherein the primary or secondary alcohol represented by General Formula (1) is ethanol.
[5] The method for producing an ester compound according to any one of [1] to [4],
   wherein the first catalyst includes a compound having at least one element selected from Periodic Table Group 10 elements, and an organic phosphine ligand.
[6] The method for producing an ester compound according to [5],
   wherein the Periodic Table Group 10 element is Pd.
[7] The method for producing an ester compound according to [5],
   wherein the organic phosphine ligand is a monodentate organic phosphine ligand.
[8] The method for producing an ester compound according to [7],
   wherein the organic phosphine ligand is an organic phosphine ligand represented by General Formula (5). 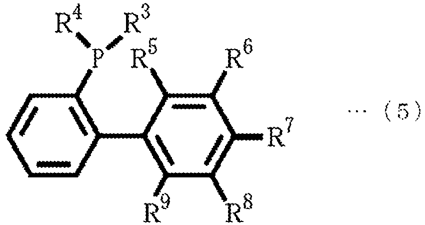 (in Formula (5), R³ and R⁴ are alkyl groups having 1 to 6 carbon atoms, cycloalkyl groups having 6 to 9 carbon atoms, or aryl groups having 6 to 9 carbon atoms, R³ and R⁴ may be the same or different from each other, R⁵ to R⁹ are hydrogen atoms, alkyl groups having 1 to 4 carbon atoms, alkoxy groups having 1 to 4 carbon atoms, or dialkylamino groups having 2 to 8 carbon atoms, and R⁵ to R⁹ may all be the same or may be partially or completely different from each other)
[9] The method for producing an ester compound according to any one of [1] to [8],
   wherein the first catalyst includes an acidic compound.
[10] The method for producing an ester compound according to [9],
   wherein the acidic compound is at least one selected from the group consisting of an alkyl sulfonic acid, an ammonium salt of an alkyl sulfonic acid, a pyridinium salt of an alkyl sulfonic acid, an aryl sulfonic acid, an ammonium salt of an aryl sulfonic acid, and a pyridinium salt of an aryl sulfonic acid.
[11] The method for producing an ester compound according to any one of [1] to [10],
   wherein the first catalyst includes a nitrogen atom-containing heterocyclic compound.
[12] The method for producing an ester compound according to [11],
   wherein the nitrogen atom-containing heterocyclic compound is at least one selected from the group consisting of pyridine and picoline.
[13] The method for producing an ester compound according to any one of [1] to [12],
   wherein the second catalyst is at least one selected from the group consisting of potassium carbonate, sodium methoxide, sodium ethoxide, sodium-t-butoxide, potassium ethoxide, tetraethoxytitanium, tetraisopropoxytitanium, tetra-n-butoxytitanium, mono-n-butyltin oxide, di-n-butyltin oxide, di-n-butyltin laurate, and di-n-octyltin oxide.
[14] The method for producing an ester compound according to any one of [1] to [13], further comprising:
   before the first step, a carbon monoxide producing step of pyrolyzing a plastic or a biomaterial to produce carbon monoxide.
[15] The method for producing an ester compound according to [4],
   wherein the ethanol is bioethanol derived from a plant.
[16] A method for producing an ester compound, comprising:
   a first step of reacting a vinyl acetate with a primary or secondary alcohol represented by General Formula (1) and carbon monoxide in a presence of a first catalyst to produce a first ester compound represented by General Formula (2),
   wherein the first catalyst includes a compound having at least one element selected from Periodic Table Group 10 elements, and an organic phosphine ligand represented by General Formula (5).
   R¹OH ··· (1)

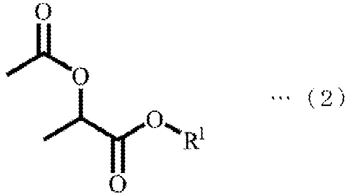

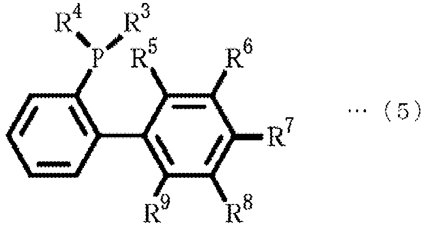

   (in Formulae (1) and (2), R¹ is a hydrocarbon group which has 1 to 10 carbon atoms and may have an alicyclic ring or an aromatic ring in part or in whole, and R¹'s in Formula (1) and Formula (2) are the same)
   (in Formula (5), R³ and R⁴ are alkyl groups having 1 to 6 carbon atoms, cycloalkyl groups having 6 to 9 carbon atoms, or aryl groups having 6 to 9 carbon atoms, R³ and R⁴ may be the same or different from each other, R⁵ to R⁹ are hydrogen atoms, alkyl groups having 1 to 4 carbon atoms, alkoxy groups having 1 to 4 carbon atoms, or dialkylamino groups having 2 to 8 carbon atoms, and R⁵ to R⁹ may all be the same or may be partially or completely different from each other)
[17] The method for producing an ester compound according to [16],
   wherein the primary or secondary alcohol represented by General Formula (1) is ethanol.
[18] The method for producing an ester compound according to [16] or [17], wherein the Periodic Table Group 10 element is Pd.
[19] The method for producing an ester compound according to any one of [16] to [18],
   wherein, in General Formula (5), R³ and R⁴ are cyclohexyl groups, R⁵ and R⁹ are methoxy groups, and R⁶ to R⁸ are hydrogen atoms.
[20] The method for producing an ester compound according to any one of [16] to [19],
   wherein the first catalyst includes an acidic compound.
[21] The method for producing an ester compound according to [20],
   wherein the acidic compound is at least one selected from the group consisting of an alkyl sulfonic acid, an ammonium salt of an alkyl sulfonic acid, a pyridinium salt of an alkyl sulfonic acid, an aryl sulfonic acid, an ammonium salt of an aryl sulfonic acid, and a pyridinium salt of an aryl sulfonic acid.
[22] The method for producing an ester compound according to any one of [16] to [21],
   wherein the first catalyst includes a nitrogen atom-containing heterocyclic compound.
[23] The method for producing an ester compound according to [22],
   wherein the nitrogen atom-containing heterocyclic compound is at least one selected from the group consisting of pyridine and picoline.

### Advantageous Effects of Invention

In the method for producing an ester compound of the present invention, a first ester compound represented by General Formula (2), which is produced by reacting a vinyl acetate with a primary or secondary alcohol represented by General Formula (1) and carbon monoxide in the presence of a first catalyst, is reacted with an alcohol in the presence of a second catalyst to produce a lactic acid ester represented by General Formula (3) and an acetic acid ester represented by General Formula (4). Therefore, according to the method for producing an ester compound of the present invention, the lactic acid ester represented by General Formula (3) and the acetic acid ester represented by General Formula (4) can be efficiently produced.

The method for producing an ester compound of the present invention includes a step of reacting a vinyl acetate with a primary or secondary alcohol represented by General Formula (1) and carbon monoxide in the presence of a first catalyst to produce a first ester compound represented by General Formula (2), in which the first catalyst includes a compound having at least one element selected from Periodic Table Group 10 elements, and an organic phosphine ligand represented by General Formula (5). As a result, according to the above-described production method, the first ester compound represented by General Formula (2) can be produced in a high yield.

### DESCRIPTION OF EMBODIMENTS

Hereinafter, the method for producing an ester compound according to the embodiment of the present invention will be described in detail. The present invention is not limited to embodiments shown below.

The method for producing an ester compound according to the present embodiment includes a first step of reacting a vinyl acetate with a primary or secondary alcohol and carbon monoxide in the presence of a first catalyst to produce a first ester compound and a second step of reacting the first ester compound with an alcohol in the presence of a second catalyst to produce a lactic acid ester and an acetic acid ester.

The method for producing an ester compound according to the present embodiment may include, before the second step, an intermediate step of reducing an unreacted vinyl acetate included in a reaction solution after the first step to convert the unreacted vinyl acetate into an acetic acid ester.

### [First step]

In the first step, a vinyl acetate is reacted with a primary or secondary alcohol represented by General Formula (1) and carbon monoxide in the presence of a first catalyst to produce a first ester compound represented by General Formula (2).

In the first step, it is preferable to carry out the reaction (alkoxycarbonylation reaction) in the first step in which the vinyl acetate, the primary or secondary alcohol represented by General Formula (1), the first catalyst, and a solvent are charged into a reaction vessel replaced with a carbon monoxide gas atmosphere.

R¹OH ··· (1)

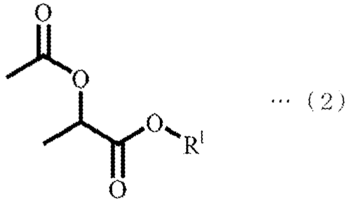

(in Formulae (1) and (2), R¹ is a hydrocarbon group which has 1 to 10 carbon atoms and may have an alicyclic ring or an aromatic ring in part or in whole, and R¹'s in Formula (1) and Formula (2) are the same)

### "Primary or secondary alcohol"

R¹ in the primary or secondary alcohol represented by General Formula (1) is a hydrocarbon group which has 1 to 10 carbon atoms and may have an alicyclic ring or an aromatic ring in part or in whole. Specific examples of the primary or secondary alcohol represented by General Formula (1) include methanol, ethanol, 1-propanol, isopropanol, 1-butanol, isobutyl alcohol, sec-butyl alcohol, 1-hexanol, 1-octanol, 2-ethylhexanol, cyclohexanemethanol, and benzyl alcohol.

In a case where the first ester compound represented by General Formula (2), in which R¹ is a methyl group, is produced by using the method for producing an ester compound according to the present embodiment, as the primary or secondary alcohol represented by General Formula (1), methanol in which R¹ is a methyl group is used.

In a case where the first ester compound represented by General Formula (2), in which R¹ is an ethyl group, is produced, as the primary or secondary alcohol represented by General Formula (1), ethanol in which R¹ is an ethyl group is used.

In a case where the first ester compound represented by General Formula (2), in which R¹ is an n-butyl group, is produced, as the primary or secondary alcohol represented by General Formula (1), 1-butanol in which R¹ is an n-butyl group is used.

Methanol, ethanol, or 1-butanol is preferable because they are highly reactive with the vinyl acetate.

As the primary or secondary alcohol represented by General Formula (1), methanol or ethanol is preferably used, and ethanol is most preferably used.

In a case where the primary or secondary alcohol represented by General Formula (1) is ethanol, as the ethanol, it is preferable to use bioethanol derived from a plant. As the bioethanol derived from a plant, a commercially available product may be used.

Examples of the bioethanol derived from a plant include those produced using edible raw materials such as corn and sugar. The corn is mainly livestock feed, and the sugar is human food.

As the bioethanol derived from a plant, those produced from biomass including lignocellulose such as wood and corn stalk as a raw material may be used. The lignocellulose is an abundant resource. The lignocellulose is very difficult to decompose as it is. Therefore, in a case where the lignocellulose is used as a raw material of the bioethanol, first, cellulose and lignin included in the lignocellulose are separated by high-temperature and high-pressure treatment or acid treatment using concentrated sulfuric acid, concentrated phosphoric acid, or the like.

Thereafter, the separated cellulose is decomposed into sugars using a special enzyme called as cellulase. The decomposed sugar may be used as a raw material for the ethanol by a normal fermentation process (see, for example, Non-Patent Document 3). In addition, another method of using the lignocellulose as a raw material for the bioethanol is a method not using chemicals. For example, there is a method of hydrolyzing biomass such as wood by contacting it with water in a subcritical or supercritical state to obtain sugars (see, for example, Patent Document 7).

In the present embodiment, as the ethanol, it is preferable to use bioethanol derived from a plant produced by using these methods.

In the method for producing an ester compound according to the present embodiment, since the first ester compound can be produced in higher yield and higher selectivity, a used amount (charged amount) of the primary or secondary alcohol represented by General Formula (1) with respect to a used amount (charged amount) of the vinyl acetate is preferably, in terms of molar ratio (vinyl acetate:primary or secondary alcohol), 1:0.5 to 1:100, preferably 1:1 to 1:10, and still more preferably 1:1.2 to 1:5.

### "Carbon monoxide"

In the present embodiment, as the carbon monoxide, it is preferable to use a gas including carbon monoxide.

The gas including carbon monoxide may be a gas including only carbon monoxide, or may be a gas including an inert gas such as nitrogen or argon, a hydrogen gas, and the like in addition to carbon monoxide. The gas including carbon monoxide preferably does not include an oxidizing gas such as air or oxygen.

In a preferred embodiment, as the carbon monoxide, it is preferable to use carbon monoxide obtained by pyrolyzing a plastic or a biomaterial. In this case, before the first step, a step (carbon monoxide producing step) of pyrolyzing a plastic or a biomaterial to produce carbon monoxide is performed.

As the plastic, it is preferable to use a waste plastic. Examples of the waste plastic include those composed of engineering plastics such as polyethylene, polypropylene, polystyrene, acrylonitrile-butadiene-styrene (ABS) resin, vinyl chloride resin, and polyethylene terephthalate (PET) resin, or thermosetting resins such as epoxy resin.

As a method for pyrolyzing a plastic to produce carbon monoxide, a waste plastic gasification process by a partial oxidation method (see, for example, Non-Patent Document 4) may be used.

Examples of the biomaterial include woody biomass including cellulose, hemicellulose, lignin, and the like, and plant-derived biomass such as vegetation-based biomass.

As a method for pyrolyzing a biomaterial to produce carbon monoxide, for example, methods described in Patent Document 8 and Patent Document 9 may be used.

In the method described in Patent Document 8, a part of waste mainly composed of organic matter is partially oxidized, and the obtained calorific value is supplied to a water gasification zone. In the water gasification zone, the remaining waste is heated to approximately 800°C to 1000°C, and gasified with steam to produce a gas having a high concentration of H₂ and CO. In this method, a gas having a high concentration of H₂ and CO can be produced without using other fuels.

In the method described in Patent Document 9, char is produced by heating waste, and steam is produced by utilizing waste heat in a waste incinerator. The char is filled in a shaft furnace and heated by energization. At the same time, the steam is supplied to the shaft furnace to produce a reformed gas consisting of carbon monoxide and hydrogen by an aqueous gasification reaction. In this method, a high-calorie and clean reformed gas which does not contain CO₂ and may be used as an alternative fuel to fossil fuels is obtained.

As a used amount of the carbon monoxide during the reaction of the first step, for example, a partial pressure in a reaction vessel with a gas atmosphere including the carbon monoxide in a case of charging a reaction solution the vinyl acetate and the primary or secondary alcohol represented by General Formula (1) is preferably in a range of 0.05 to 50 MPa, preferably in a range of 0.1 to 30 MPa, and still more preferably in a range of 1 to 6 MPa. It is particularly preferable to perform the reaction while supplementing the carbon monoxide consumed by the reaction so that the partial pressure of carbon monoxide in the above-described reaction vessel during the reaction is maintained in the range of 1 to 6 MPa. In a case where the partial pressure of carbon monoxide in the above-described reaction vessel during the reaction is 0.05 MPa or greater, the reaction proceeds smoothly. It is preferable that the partial pressure of carbon monoxide in the above-described reaction vessel during the reaction is high in order to promote the reaction. However, in order to increase the partial pressure of carbon monoxide during the reaction to greater than 50 MPa, it is necessary to use an expensive device. By setting the partial pressure of carbon monoxide during the reaction to 50 MPa or less, the first ester compound represented by General Formula (2) can be produced by using an industrially suitable device and method.

### "First ester compound"

R¹ in the first ester compound represented by General Formula (2) is the same as R¹ in the primary or secondary alcohol represented by General Formula (1). Since the first ester compound is produced in high yield and high selectivity by performing the first step, R¹ in the first ester compound is preferably any one selected from a methyl group, an ethyl group, and an n-butyl group, and more preferably a methyl group or an ethyl group.

### "First catalyst"

The first catalyst preferably includes a compound having at least one element selected from Periodic Table Group 10 elements, and an organic phosphine ligand.

The first catalyst more preferably includes an acidic compound. In the present specification, the term "acidic compound" means Bronsted acid, a salt of Bronsted acid, or Lewis acid. The acidic compound is preferably Bronsted acid or a salt of Bronsted acid.

In addition, it is more preferable that the first catalyst includes a nitrogen atom-containing heterocyclic compound.

In the present embodiment, in a case where the acidic compound used as the first catalyst is a compound having a nitrogen atom-containing heterocycle, the acidic compound can also serve as a nitrogen atom-containing heterocyclic compound. Examples of the acidic compound which also serves as a nitrogen atom-containing heterocyclic compound include picolinic acid.

Examples of the compound having at least one element selected from Periodic Table Group 10 elements include compounds including at least one element selected from Ni, Pd, and Pt.

Examples of the compound including nickel (Ni) include at least one kind of zero-valent or divalent nickel compounds selected from nickel (II) chloride, nickel (II) nitrate, nickel (II) sulfate, and nickel (II) acetate.

Examples of the compound including palladium (Pd) include at least one kind of zero-valent or divalent palladium compounds selected from palladium (II) chloride, palladium (II) nitrate, palladium (II) sulfate, palladium (II) acetate, bis(acetylacetonato)palladium (II) (Pd(acac)₂), tris(dibenzylideneacetone)dipalladium (0) (Pd₂(dba)₃), and dipalladium(0) tris(dibenzylideneacetone)chloroform (Pd₂(dba)₃·CHCl₃).

Examples of the compound including platinum (Pt) include tetrachloroplatinic (II) acid, hexachloroplatinic (IV) acid, platinum (II) acetate, and platinum (IV) sulfate.

Among these compounds having at least one element selected from Periodic Table Group 10 elements, in particular, it is preferable to use a compound including palladium. This is because that the methoxycarbonylation reaction for producing the first ester compound represented by General Formula (2) can be effectively promoted, so that reaction conditions of the first step can be set to mild conditions.

Among these compounds having at least one element selected from Periodic Table Group 10 elements, from the viewpoint of reaction rate and selectivity, it is preferable to use a compound including palladium, and it is particularly preferable to use palladium (II) chloride, palladium (II) nitrate, or bis(acetylacetonato)palladium (II) (Pd(acac)₂).

The compound having at least one element selected from Periodic Table Group 10 elements, which is included in the first catalyst, may be only one kind or two or more kinds thereof.

A used amount of the compound having at least one element selected from Periodic Table Group 10 elements is preferably 0.00001 to 0.2 molar parts, more preferably 0.0001 to 0.1 molar parts, and still more preferably 0.001 to 0.05 molar parts with respect to 1 molar part of the vinyl acetate. In a case where the used amount of the compound having at least one element selected from Periodic Table Group 10 elements is 0.00001 molar parts or greater with respect to 1 molar part of the vinyl acetate, a concentration of reactive species (hydride complex and the like) of the catalyst can be secured. Therefore, the alkoxycarbonylation reaction of producing the first ester compound represented by General Formula (2) is effectively promoted. In addition, in a case where the used amount of the compound having at least one element selected from Periodic Table Group 10 elements is 0.2 molar part or less, it is economically preferable.

The organic phosphine ligand coordinates to an element included in the compound having at least one element selected from Periodic Table Group 10 elements. The organic phosphine ligand is preferably a monodentate organic phosphine ligand, and more preferably an organic phosphine ligand represented by General Formula (5).

In a case where the first catalyst includes the compound having at least one element selected from Periodic Table Group 10 elements and the organic phosphine ligand represented by General Formula (5), the first ester compound represented by General Formula (2) can be produced in high yield. 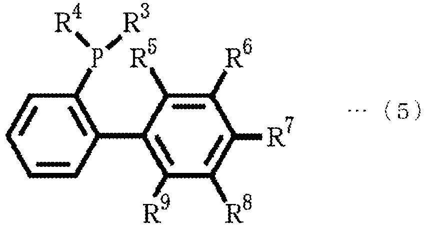 (in Formula (5), R³ and R⁴ are alkyl groups having 1 to 6 carbon atoms, cycloalkyl groups having 6 to 9 carbon atoms, or aryl groups having 6 to 9 carbon atoms, R³ and R⁴ may be the same or different from each other, R⁵ to R⁹ are hydrogen atoms, alkyl groups having 1 to 4 carbon atoms, alkoxy groups having 1 to 4 carbon atoms, or dialkylamino groups having 2 to 8 carbon atoms, and R⁵ to R⁹ may all be the same or may be partially or completely different from each other)

R³ and R⁴ in the organic phosphine ligand represented by General Formula (5) alkyl groups having 1 to 6 carbon atoms, cycloalkyl groups having 6 to 9 carbon atoms, or aryl groups having 6 to 9 carbon atoms. R³ and R⁴ may be the same or different from each other. Since the first ester compound can be produced in higher yield and higher selectivity, R³ and R⁴ are preferably cyclohexyl groups, and in particular, both R³ and R⁴ are preferably cyclohexyl groups.

R⁵ to R⁹ in the organic phosphine ligand represented by General Formula (5) are hydrogen atoms, alkyl groups having 1 to 4 carbon atoms, alkoxy groups having 1 to 4 carbon atoms, or dialkylamino groups having 2 to 8 carbon atoms. R⁵ to R⁹ may all be the same, or may be partially or completely different from each other. In particular, since the first ester compound can be produced in higher yield and higher selectivity, it is preferable that R⁵ and R⁹ are methoxy groups and R⁶ to R⁸ are hydrogen atoms.

Specific examples of the monodentate organic phosphine ligand include triphenylphosphine, tri-o-tolylphosphine, tri-m-tolylphosphine, tri-p-tolylphosphine, tri-2,4-xylylphosphine, tri-2,5-xylylphosphine, tri-3,5-xylylphosphine, tris(p-tert-butylphenyl)phosphine, tris(p-methoxyphenyl)phosphine, tris(o-methoxyphenyl)phosphine, tris(p-tert-butoxyphenyl)phosphine, diphenyl-2-pyridylphosphine, diphenylcyclohexylphosphine, tricyclohexylphosphine, tri-n-butylphosphine, tri-n-octylphosphine, tri-tert-butylphosphine, di-tert-butyl(2-butenyl)phosphine, di-tert-butyl(3-methyl-2-butenyl)phosphine, di-tert-butylphenylphosphine, SPhos represented by Formula (5-1), XPhos represented by Formula (5-4), MePhos represented by Formula (5-5), RuPhos represented by Formula (5-6), organic phosphine ligands represented by Formulae (5-2) and (5-3), DavePhos, JohnPhos, BrettPhos, CPhos, and AlPhos. 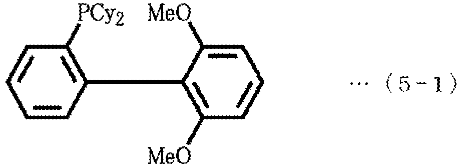 (in Formula (5-1), Cy is a cyclohexyl group and Me is a methyl group) 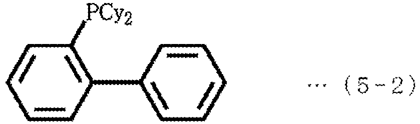 (in Formula (5-2), Cy is a cyclohexyl group) 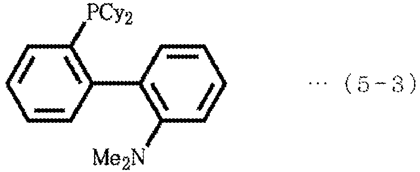 (in Formula (5-3), Cy is a cyclohexyl group and Me is a methyl group) 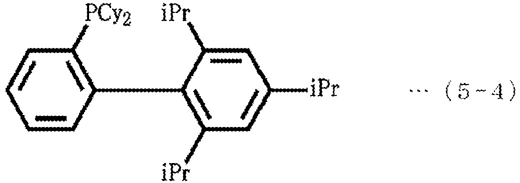 (in Formula (5-4), Cy is a cyclohexyl group and iPr is an isopropyl group) 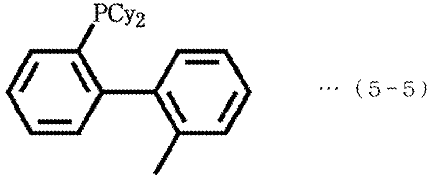 (in Formula (5-5), Cy is a cyclohexyl group) 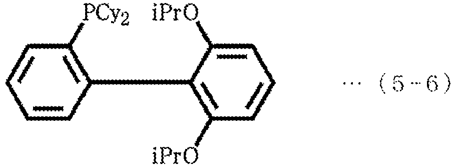 (in Formula (5-6), Cy is a cyclohexyl group and iPr is an isopropyl group)

As the organic phosphine ligand represented by General Formula (5), in particular, since the first ester compound can be produced in higher yield and higher selectivity, it is preferable to use the organic phosphine ligand represented by Formula (5-1). In the organic phosphine ligand represented by Formula (5-1), R³ and R⁴ in General Formula (5) are cyclohexyl groups, R⁵ and R⁹ are methoxy groups, and R⁶ to R⁸ are hydrogen atoms.

The organic phosphine ligand included in the first catalyst may be only one kind or two or more kinds thereof.

A used amount of the organic phosphine ligand is preferably 0.5 to 5 molar parts, more preferably 1 to 4 molar parts, and still more preferably 1.5 to 3 molar parts with respect to 1 molar part of the compound having at least one element selected from Periodic Table Group 10 elements. In a case where the used amount of the organic phosphine ligand is 0.5 molar parts or greater with respect to 1 molar part of the compound having at least one element selected from Periodic Table Group 10 elements, the reactive species of the catalyst, to which organic phosphine is effectively coordinated, are effectively produced. As a result, the alkoxycarbonylation reaction is promoted, and the first ester compound is produced with higher selectivity.

In a case where the used amount of the organic phosphine ligand is 5 molar parts or less with respect to 1 molar part of the compound having at least one element selected from Periodic Table Group 10 elements, the reaction for producing the first ester compound can be effectively promoted. This is because that, in a case where the organic phosphine ligands are too many, in the alkoxycarbonylation reaction of producing the first ester compound, carbon monoxide is difficult to coordinate with the Periodic Table Group 10 element, so that it is possible to prevent the reaction rate of the alkoxycarbonylation reaction from slowing down.

The acidic compound promotes the alkoxycarbonylation reaction of producing the first ester compound. The acidic compound functions as a hydride agent for at least one element selected from the Periodic Table Group 10 elements. As a result, it is presumed that the alkoxycarbonylation reaction is promoted.

Examples of the acidic compound include hydrochloric acid, nitric acid, sulfuric acid, alkanoic acid having 2 to 12 carbon atoms, sulfonic acids such as methanesulfonic acid, chlorosulfonic acid, fluorosulfonic acid, trifluoromethanesulfonic acid, benzenesulfonic acid, naphthalenesulfonic acid, o-toluenesulfonic acid, m-toluenesulfonic acid, p-toluenesulfonic acid, t-butylsulfonic acid, and 2-hydroxypropanesulfonic acid, and a salt thereof, sulfonated ion exchange resin, perhalogen acids such as perchloric acid, perfluorocarboxylic acids such as trichloroacetic acid and trifluoroacetic acid, phosphonic acids such as orthophosphoric acid and benzenephosphonic acid, and esters of sulfonic acid, such as dimethyl sulfate, methyl methanesulfonate, and methyl trifluoromethanesulfonate.

Among these, since acidity is appropriate, the acidic compound is preferably at least one selected from the group consisting of an alkyl sulfonic acid, an ammonium salt of an alkyl sulfonic acid, a pyridinium salt of an alkyl sulfonic acid, an aryl sulfonic acid, an ammonium salt of an aryl sulfonic acid, and a pyridinium salt of an aryl sulfonic acid, and particularly preferably p-toluenesulfonic acid.

In a case where an acid strength of the first catalyst is too strong, a side reaction between the vinyl acetate which is a raw material and the primary or secondary alcohol represented by General Formula (1) is likely to occur. In addition, in a case where the acid strength of the first catalyst is too weak, the effect of increasing the reaction rate of the alkoxycarbonylation reaction of producing the first ester compound may be insufficient.

The acidic compound included in the first catalyst may be only one kind or two or more kinds thereof.

A used amount of the acidic compound is preferably in a range of 0.05 to 4 molar parts, more preferably in a range of 0.1 to 2 molar parts, and still more preferably 0.2 to 1 molar part of an acid point (proton) of the acidic compound with respect to 1 molar part of the compound having at least one element selected from Periodic Table Group 10 elements. In a case where the acid point of the acidic compound is 0.05 molar parts or greater with respect to 1 molar part of the compound having at least one element selected from Periodic Table Group 10 elements, the reactive species (hydride complex and the like) of the catalyst are efficiently produced. Therefore, the alkoxycarbonylation reaction of producing the first ester compound is effectively promoted. In addition, in a case where the acid point of the acidic compound is 4 molar parts or less with respect to 1 molar part of the compound having at least one element selected from Periodic Table Group 10 elements, side reactions and the like in the alkoxycarbonylation reaction can be effectively suppressed.

In a case where the first catalyst includes the nitrogen atom-containing heterocyclic compound together with the acidic compound, the activity of the first catalyst is improved, and the alkoxycarbonylation reaction of producing the first ester compound is further promoted. It is presumed that this is due to the following reasons. The nitrogen atom-containing heterocyclic compound has a nitrogen atom-containing heterocycle. Therefore, in a case where the nitrogen atom-containing heterocyclic compound is included in the first catalyst together with the acidic compound, the acidity of the first catalyst can be easily adjusted within a range in which the alkoxycarbonylation reaction is promoted. In addition, for example, in a case where a chloride of at least one element selected from the Periodic Table Group 10 elements (palladium chloride and the like) is used as the compound having at least one element selected from Periodic Table Group 10 elements, and a basic nitrogen atom-containing heterocyclic compound is used, decomposition of the chloride (palladium chloride and the like) is suppressed, and hydrochloric acid generated by the decomposition of the chloride is neutralized, thereby suppressing side reactions. As a result, the first catalyst is activated, it is presumed that the alkoxycarbonylation reaction is promoted.

Examples of the nitrogen atom-containing heterocyclic compound include pyridine, picoline, imidazole, pyrazole, and oxazole. Among these, the nitrogen atom-containing heterocyclic compound is preferably at least one selected from the group consisting of pyridine and picoline, and more preferably pyridine. This is because that the first ester compound can be produced in higher yield and higher selectivity.

The nitrogen atom-containing heterocyclic compound included in the first catalyst may be only one kind or two or more kinds thereof.

A used amount of the nitrogen atom-containing heterocyclic compound is preferably 0.1 to 50 molar parts, more preferably 1 to 30 molar parts, and still more preferably 3 to 20 molar parts with respect to 1 molar part of the compound having at least one element selected from Periodic Table Group 10 elements. In a case where the used amount of the nitrogen atom-containing heterocyclic compound is 0.1 molar parts or greater with respect to 1 molar part of the compound having at least one element selected from Periodic Table Group 10 elements, the acidity of the first catalyst tends to be within the range in which the alkoxycarbonylation reaction is promoted, and the first ester compound is likely to be produced with high selectivity. In addition, in a case where the used amount is 50 molar parts or less with respect to 1 molar part of the compound having at least one element selected from Periodic Table Group 10 elements, side reactions are suppressed, which is preferable.

### "Solvent"

In the first step, a solvent may be used as necessary. As the solvent used in the first step, it is preferable to use a chemical species which does not participate in the reaction between the vinyl carboxylate, the primary or secondary alcohol, and carbon monoxide. Examples of such a solvent include tetrahydrofuran, dioxane, acetone, methyl ethyl ketone, cyclohexanone, ethyl acetate, n-propyl acetate, n-butyl acetate, n-heptane, acetonitrile, toluene, xylene, N-methylpyrrolidone, γ-butyrolactone, N,N-dimethylformamide, and N,N-dimethylacetamide. In addition, it is also possible to use the primary or secondary alcohol itself, which is the reaction substrate, as the solvent.

Among these solvents, in particular, since the first ester compound can be produced in higher yield and higher selectivity, it is preferable to use any one or two or more kinds selected from tetrahydrofuran, acetone, ethyl acetate, toluene, and xylene, and from the viewpoint of solubility of the catalyst and ease of separation from the product, it is particularly preferable to use toluene.

As a used amount of the solvent, a concentration of the vinyl acetate in the reaction solution is preferably in a range of 0.05 to 20 mol/liter, more preferably in a range of 0.1 to 10 mol/liter, and still more preferably in a range of 0.5 to 5 mol/liter. In a case where the concentration of the vinyl acetate in the reaction solution is 0.05 mol/liter or greater, the concentration of the vinyl acetate in the reaction solution tends to be within the range in which the alkoxycarbonylation reaction is promoted, and the first ester compound is likely to be produced with high selectivity. In a case where the concentration of the vinyl acetate in the reaction solution is 20 mol/liter or less, it is easy to secure a substrate concentration which allows the reaction to proceed smoothly, which is preferable.

Reaction conditions of the first step can be appropriately determined according to the type and the like of the first ester compound to be produced within a range in which the alkoxycarbonylation reaction of the vinyl acetate, the primary or secondary alcohol, and carbon monoxide gas proceeds.

For example, It is preferable that the reaction conditions satisfy one or more of (a) reaction pressure, (b) reaction temperature, and (c) reaction time described below.

### (a) Reaction pressure

In the first step, for example, the partial pressure of the carbon monoxide in the reaction vessel is preferably 0.05 to 50 MPa, more preferably 0.1 to 30 MPa, and still more preferably 1 to 6 MPa. By setting the partial pressure of carbon monoxide in the reaction vessel to 0.05 to 50 MPa, the first ester compound can be produced in higher yield and higher selectivity.

### (b) Reaction temperature

In the first step, for example, the temperature inside the reaction vessel is preferably 60°C to 140°C and more preferably 80°C to 120°C. In a case where the temperature inside the reaction vessel is 60°C or higher, the alkoxycarbonylation reaction of producing the first ester compound is further promoted. In a case where the temperature inside the reaction vessel is 140°C or lower, side reactions in the alkoxycarbonylation reaction can be suppressed, which is preferable.

### (c) Reaction time

For example, the first step is performed for preferably 1 to 40 hours and more preferably 10 to 20 hours. In a case where the reaction time in the first step is 1 hour or greater, the first ester compound can be produced in higher yield and higher selectivity. In a case where the reaction time in the first step is 40 hours or less, side reactions associated with the production of the first ester compound can be suppressed.

### [Intermediate step]

Next, the intermediate step will be described. The intermediate step is a step performed after the first step and before the second step, as necessary. It is not necessary to perform the intermediate step.

In the intermediate step, an unreacted vinyl acetate included in the reaction solution after the first step is reduced to convert the unreacted vinyl acetate into an acetic acid ester.

As a method for reducing the vinyl acetate, for example, a hydrogen reduction method in which the vinyl acetate is reacted with hydrogen gas in the presence of a hydrogen reduction catalyst to convert the vinyl acetate into an acetic acid ester. By using the hydrogen reduction method, the vinyl acetate can be reduced efficiently and at low cost.

The reduction reaction of the vinyl acetate in the intermediate step is preferably carried out in a reaction vessel having a hydrogen gas atmosphere by charging a solution including the unreacted vinyl acetate included in the reaction solution after the first step.

As the solution including the unreacted vinyl acetate, the reaction solution after the first step may be used as it is, a solution obtained by adding a solvent to the reaction solution after the first step may be used, or a solution in which vinyl acetate isolated from the reaction solution after the first step is dissolved in a solvent may be used.

As a method for isolating the vinyl acetate from the reaction solution after the first step, known methods such as distillation, extraction, and column chromatographic purification may be used alone or a combination of two or more may be used.

In a case where the solution obtained by adding a solvent to the reaction solution after the first step or the solution in which vinyl acetate isolated from the reaction solution after the first step is dissolved in a solvent is used as the solution including the unreacted vinyl acetate, as the solvent, for example, tetrahydrofuran, dioxane, acetone, methyl ethyl ketone, cyclohexanone, ethyl acetate, n-propyl acetate, n-butyl acetate, ethyl lactate, ethyl 2-acetoxypropionate, n-heptane, acetonitrile, toluene, xylene, N-methylpyrrolidone, γ-butyrolactone, N,N-dimethylformamide, N,N-dimethylacetamide, or the like may be used. Among these solvents, in particular, since a high yield can be obtained, any one selected from toluene, xylene, the first ester compound represented by General Formula (2), which is the product in the first step, and the lactic acid ester represented by General Formula (3) and the acetic acid ester represented by General Formula (4) described later, which are products in the second step, can be preferably used.

The reduction reaction of the vinyl acetate in the intermediate step is preferably carried out using, as the solution including the unreacted vinyl acetate, a solution in which the concentration of the vinyl acetate is 0.1% to 70% by mass.

As the catalyst used for the reduction reaction of reducing the vinyl acetate, it is preferable to use a catalyst having high hydrogen reduction reaction activity of a carbon-carbon double bond.

In a case where, as the solution including the unreacted vinyl acetate, the reaction solution after the first step is used as it is or the solution obtained by adding a solvent to the reaction solution after the first step is used, the first catalyst used in the first step may be used as the catalyst for reducing the vinyl acetate, or a catalyst having high hydrogen reduction reaction activity of a carbon-carbon double bond may be used together with the first catalyst.

Examples of the catalyst having high hydrogen reduction reaction activity of a carbon-carbon double bond include a catalyst in which a noble metal (Pd, Rh, Pt, Ru, and the like) is supported on a sponge nickel, sponge cobalt, or carrier (activated carbon, silica, alumina, and the like). Among these catalysts, it is preferable to use a catalyst in which palladium is supported on a carrier such as activated carbon and silica.

In a case where a catalyst in which a noble metal is supported on a carrier consisting of activated carbon or silica is used, side reactions are unlikely to occur in the reduction reaction of reducing the vinyl acetate, and separation from the reaction solution after the reduction reaction is easy.

A supported amount of the noble metal supported in the catalyst on which the noble metal is supported on the above-described carrier is preferably 0.1 to 15 parts by mass, more preferably 0.5 to 12 parts by mass, and still more preferably 1 to 10 parts by mass with respect to 100 parts by mass of the carrier.

The catalyst having high hydrogen reduction reaction activity of a carbon-carbon double bond is preferably used in an amount of 0.01 to 20 parts by mass, more preferably used in an amount of 0.5 to 15 parts by mass, and still more preferably used in an amount of 1 to 10 parts by mass with respect to 100 parts by mass of the unreacted vinyl acetate.

In a case where the catalyst in which palladium is supported on the carrier consisting of activated carbon or silica is used as the catalyst having high hydrogen reduction reaction activity of a carbon-carbon double bond, the catalyst can be easily recovered by filtering the reaction solution after the reduction reaction. In addition, the recovered catalyst in which palladium is supported on the carrier consisting of activated carbon or silica can again be used as the catalyst in the reduction reaction of reducing the vinyl acetate. Therefore, the catalyst in which palladium is supported on the carrier consisting of activated carbon or silica promotes the reduction reaction of the vinyl acetate, so that it may be preferable to use the catalyst in excess with respect to the vinyl acetate to shorten the reaction time.

In the intermediate step, for example, a reaction temperature of the reaction of reducing the unreacted vinyl acetate is preferably room temperature (20°C) to 160°C and more preferably 20°C to 120°C. By setting the reaction temperature in the reaction of reducing the vinyl acetate to room temperature or higher, the reduction reaction can be promoted. In addition, by setting the reaction temperature to 160°C or lower, side reactions in the reduction reaction of reducing the vinyl acetate can be suppressed.

In the reaction of reducing the unreacted vinyl acetate of the intermediate step, the pressure of the hydrogen gas inside the reaction vessel which includes the solution including the unreacted vinyl acetate is set to preferably normal pressure (0.1 MPa) to 1.0 MPa, more preferably 0.1 MPa to 0.8 MPa, and still more preferably 0.1 MPa to 0.5 MPa. By setting the pressure of the hydrogen gas inside the reaction vessel to normal pressure (0.1 MPa) to 1.0 MPa, the vinyl acetate can be reduced more efficiently.

### [Second step]

In the second step, the first ester compound represented by General Formula (2) is reacted with an alcohol in the presence of a second catalyst to produce a lactic acid ester represented by General Formula (3) and an acetic acid ester represented by General Formula (4). 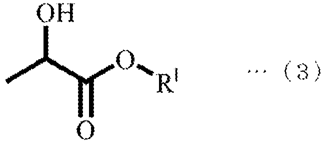 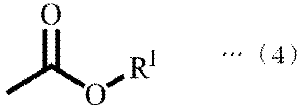 (in Formulae (3) and (4), R¹ is a hydrocarbon group which has 1 to 10 carbon atoms and may have an alicyclic ring or an aromatic ring in part or in whole, and R¹'s in Formula (3) and Formula (4) are the same)

In the second step, it is preferable to use a solution including the first ester compound represented by General Formula (2). As the solution including the first ester compound, the reaction solution after the first step or the reaction solution after the intermediate step may be used as it is, a solution obtained by adding a solvent to the reaction solution after the first step or the reaction solution after the intermediate step may be used, or a solution in which the first ester compound isolated from the reaction solution after the first step or the reaction solution after the intermediate step is dissolved in a solvent may be used. In a case where a conversion rate of the vinyl acetate in the first step is low, in the second step, as the solution including the first ester compound, the solution in which the first ester compound isolated from the reaction solution after the first step or the reaction solution after the intermediate step is dissolved in a solvent is preferably used.

As a method for isolating the first ester compound from the reaction solution after the first step or the reaction solution after the intermediate step, known methods such as distillation, extraction, and column chromatographic purification may be used alone or a combination of two or more may be used.

### "Solvent"

In the second step, the alcohol used in the reaction can also serve as the solvent. Therefore, it is not necessary to use a solvent in the second step. In the second step, in a case where, as the solution including the first ester compound, the reaction solution after the first step or the reaction solution after the intermediate step is used, or a case where, as the solution including the first ester compound, the solution obtained by adding a solvent to the reaction solution after the first step or the reaction solution after the intermediate step is used, the solvent included in the reaction solution after the first step or the reaction solution after the intermediate step may be used as it is as the solvent in the second step.

In the second step, in a case where a solvent is used separately from the alcohol, it is preferable to use a solvent which is inactive against the reaction between the first ester compound and the alcohol. As such a solvent, for example, any one selected from toluene, xylene, the first ester compound represented by General Formula (2), which is the product in the first step, and the lactic acid ester represented by General Formula (3) and the acetic acid ester represented by General Formula (4), which are products in the second step, can be preferably used.

The alcohol used in the second step may be the same as or different from the primary or secondary alcohol represented by General Formula (1) used in the first step. Since the used amount of alcohol in the second step is small, it is preferable that the alcohol used in the second step is the same as the primary or secondary alcohol represented by General Formula (1).

In a case where the alcohol used in the second step is the same as the primary or secondary alcohol represented by General Formula (1), the reaction between the first ester compound and the alcohol in the second step is represented by the following formula. 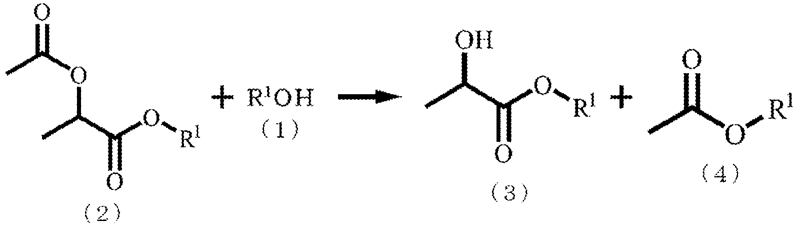 (in Formulae (1) to (4), R¹ is a hydrocarbon group which has 1 to 10 carbon atoms and may have an alicyclic ring or an aromatic ring in part or in whole, and R¹'s in Formulae (1) to (4) are the same)

In a case where the alcohol used in the second step is the same as the primary or secondary alcohol represented by General Formula (1), it is preferable that both are ethanol. In a case where both are ethanol, a produced amount of the acetic acid ester represented by General Formula (4) obtained after the second step is increased by performing the intermediate step. That is, in a case where both are ethanol, the acetic acid ester represented by General Formula (4) produced in the second step is ethyl acetate. In addition, in a case of reducing the unreacted vinyl acetate in the intermediate step, ethyl acetate is produced. Therefore, the produced amount of the acetic acid ester represented by General Formula (4) obtained after the second step is the sum of the produced amount in the second step and the produced amount in the intermediate step. Accordingly, the acetic acid ester represented by General Formula (4) (ethyl acetate) can be produced in a high yield.

In a case where the unreacted vinyl acetate is included in the reaction solution after the first step, since a boiling point of the vinyl acetate is close to a boiling point of the ethyl acetate (boiling point of vinyl acetate: 72°C, boiling point of ethyl acetate: 77°C), separation of the vinyl acetate from the reaction solution after the first step requires a complicated purification process, which is not preferable.

In a case where the alcohol used in the second step is the same as the primary or secondary alcohol represented by General Formula (1), the alcohol used in the second step is preferably 1 molar part or greater and more preferably 1.5 to 3 molar parts with respect to 1 molar part of the first ester compound represented by General Formula (2).

In a case where the alcohol used in the second step is the same as the primary or secondary alcohol represented by General Formula (1) and the reaction solution after the first step or the reaction solution after the intermediate step is used as the solution including the first ester compound, for example, in a case where the primary or secondary alcohol represented by General Formula (1) remains in the reaction solution after the first step or the reaction solution after the intermediate step, it may be used as it is as a raw material in the second step. In a case where the molar amount of the remaining primary or secondary alcohol represented by General Formula (1) is less than the molar amount of the first ester compound, it is preferable to add the primary or secondary alcohol represented by General Formula (1) so that the amount is equal to or more than the molar amount of the first ester compound.

In addition, in a case where the alcohol used in the second step is the same as the primary or secondary alcohol represented by General Formula (1) and the solution including the first ester compound isolated from the reaction solution after the first step or the reaction solution after the intermediate step is used as the solution including the first ester compound, the alcohol used in the second step is preferably 1 to 100 molar parts, more preferably 1.2 to 50 molar parts, and still more preferably 1.5 to 10 molar parts with respect to 1 molar part of the isolated first ester compound.

In a case where the alcohol used in the second step is different from the primary or secondary alcohol represented by General Formula (1), the reaction between the first ester compound represented by General Formula (2) and the alcohol in the second step is represented by the following formula. 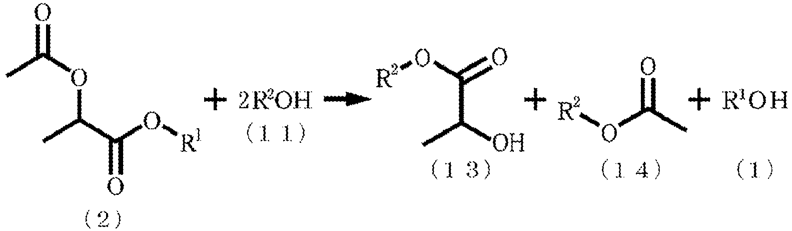 (in Formulae (1) and (2), R¹ is a hydrocarbon group which has 1 to 10 carbon atoms and may have an alicyclic ring or an aromatic ring in part or in whole, and R¹'s in Formulae (1) and (2) are the same; in Formulae (11), (13), and (14), R² is a hydrocarbon group which has 1 to 10 carbon atoms and may have an alicyclic ring or an aromatic ring in part or in whole, in which the hydrocarbon group is different from R¹, and R²'s in Formulae (11), (13), and (14) are the same)

In a case where the alcohol used in the second step is different from the primary or secondary alcohol represented by General Formula (1), as represented by the above formula, the alcohol represented by General Formula (11) is used both for producing the lactic acid ester represented by General Formula (13) and for producing the acetic acid ester represented by General Formula (14). Therefore, the alcohol represented by General Formula (11) is used preferably 2 to 200 molar parts, more preferably 2.5 to 100 molar parts, and still more preferably 3 to 10 molar parts with respect to 1 molar part of the first ester compound represented by General Formula (2).

In a case where the alcohol used in the second step is different from the primary or secondary alcohol represented by General Formula (1), as the alcohol used in the second step, it is preferable to use an alcohol having a boiling point greater than that of the primary or secondary alcohol represented by General Formula (1). In this case, in the second step, since the primary or secondary alcohol represented by General Formula (1) is removed by reactive distillation, the chemical equilibrium can be changed to increase the conversion rate of the first ester compound. In addition, in a case where an alcohol having a boiling point greater than that of the primary or secondary alcohol represented by General Formula (1) is used as the alcohol used in the second step, before reacting the first ester compound with the alcohol the primary or secondary alcohol represented by General Formula (1) may be removed by a method such as distillation. As a result, the lactic acid ester represented by General Formula (13) and the acetic acid ester represented by General Formula (14) can be efficiently produced.

### "Second catalyst"

Examples of the second catalyst used in the second step include an acid catalyst, a base catalyst, and a metal compound. Examples of the acid catalyst include sulfuric acid, p-toluenesulfonic acid, and methanesulfonic acid. Examples of the base catalyst include sodium hydroxide, potassium hydroxide, calcium hydroxide, sodium carbonate, potassium carbonate, potassium acetate, and alkali metal alkoxide. Examples of the metal compound include compounds including a metal such as titanium, tin, zinc, lead, manganese, and cobalt.

Among the above, as the second catalyst, in particular, since a moderate reaction rate can be obtained, it is preferable to use at least one selected from the group consisting of potassium carbonate, alkoxides of alkali metal (sodium methoxide, sodium ethoxide, sodium-t-butoxide, potassium ethoxide, and the like), titanium-based catalysts (tetraethoxytitanium, tetraisopropoxytitanium, tetra-n-butoxytitanium, and the like), and tin-based catalysts (mono-n-butyltin oxide, di-n-butyltin oxide, di-n-butyltin laurate, din-octyltin oxide, and the like).

A used amount of the second catalyst is preferably 0.01 to 1000 parts by mass, more preferably 0.1 to 150 parts by mass, and still more preferably 0.5 to 50 parts by mass with respect to 100 parts by mass of the first ester compound represented by Formula (2).

In a case of industrially producing the ester compound using the method for producing an ester compound according to the present embodiment, a used amount of the second catalyst is preferably 0.01 to 10 parts by mass, more preferably 0.1 to 5 parts by mass, and still more preferably 0.5 to 2 parts by mass with respect to 100 parts by mass of the first ester compound represented by Formula (2).

The reaction between the first ester compound and the alcohol in the second step is preferably carried out while distilling off the acetic acid ester represented by General Formula (4), which is produced by the reaction, to change the chemical equilibrium. This is to efficiently produce the lactic acid ester represented by General Formula (3) and the acetic acid ester represented by General Formula (4). In a case where the produced acetic acid ester represented by General Formula (4) is azeotropically heated with the alcohol, it is desirable to supplement the reaction system with the alcohol lost by distilling off the acetic acid ester represented by General Formula (4). This is to efficiently produce the lactic acid ester represented by General Formula (3) and the acetic acid ester represented by General Formula (4).

Reaction conditions of the second step can be appropriately determined according to the type and the like of the lactic acid ester represented by General Formula (3) and the acetic acid ester represented by General Formula (4) to be produced within a range in which the transesterification reaction between the first ester compound represented by General Formula (2) and the alcohol proceeds.

For example, it is preferable that the reaction conditions satisfy one or more of (d) reaction pressure, (e) reaction temperature, and (f) reaction time described below.

### (d) Reaction pressure

In the second step, for example, the pressure inside the reaction vessel is preferably normal pressure (0.1 MPa) to 0.5 MPa and more preferably 0.1 to 0.3 MPa. By setting the pressure inside the reaction vessel to 0.1 to 0.5 MPa, the acetic acid ester represented by General Formula (4) produced in the second step can be easily distilled off. In addition, in a case where the pressure inside the reaction vessel is 0.1 to 0.5 MPa, the reaction temperature can be easily controlled, and the second catalyst can be prevented from being inactivated at a high temperature.

### (e) Reaction temperature

In the second step, for example, the temperature inside the reaction vessel is preferably 20°C to 200°C, more preferably 60°C to 180°C, and still more preferably 80°C to 150°C. In a case where the temperature inside the reaction vessel is 20°C or higher, the transesterification reaction between the first ester compound represented by General Formula (2) and the alcohol is further promoted. In a case where the temperature inside the reaction vessel is 200°C or lower, side reactions in the second step can be suppressed, which is preferable.

### (f) Reaction time

For example, the second step is performed for preferably 0.5 to 48 hours, more preferably 1 to 24 hours, and still more preferably 2 to 12 hours. In a case where the reaction time in the second step is 0.5 hours or greater, the transesterification reaction between the first ester compound represented by General Formula (2) and the alcohol can be sufficiently proceeded. In a case where the reaction time in the reaction step is 48 hours or less, side reactions associated with the production of the lactic acid ester represented by General Formula (3) and the acetic acid ester represented by General Formula (4) can be suppressed.

The method for producing an ester compound according to the embodiment of the present invention is not limited to the production method described in the above-described embodiments.

For example, in the method for producing an ester compound according to the embodiment of the present invention, acetaldehyde may be produced as a by-product in the first step. In this case, it is preferable to hydrogenate the acetaldehyde included in the reaction solution after the first step and convert the acetaldehyde into ethanol by performing the above-described intermediate step. Further, in the second step, it is preferable that the first ester compound in the reaction solution after the intermediate step is reacted with the ethanol produced in the intermediate step in the presence of the second catalyst. In a case of producing the ethyl lactate in the second step, the ethanol produced from the acetaldehyde in the intermediate step may be used as a raw material. As a result, the used amount of the alcohol (ethanol) as a raw material in the second step can be reduced.

The boiling point of the acetaldehyde (21°C) is sufficiently lower than that of the lactic acid ester represented by General Formula (3) produced in the second step (for example, methyl lactate (boiling point: 145°C) and ethyl lactate (boiling point: 154°C)) and the acetic acid ester represented by General Formula (4) (for example, methyl acetate (boiling point: 57°C) and ethyl acetate (boiling point: 77°C)). Therefore, even in a case where the acetaldehyde is produced as a by-product in the first step, it is easy to recover the acetaldehyde after the second step. However, even in a case where the acetaldehyde is recovered after the second step, the acetaldehyde cannot be used as a raw material in the method for producing an ester compound according to the present embodiment. Accordingly, it is preferable to hydrogenate the acetaldehyde included in the reaction solution after the first step and convert the acetaldehyde into ethanol by performing the above-described intermediate step, thereby using the acetaldehyde as a raw material in the intermediate step.

### EXAMPLES

Hereinafter, the present invention will be described in more detail with reference to Examples and Comparative Examples. The present invention is not limited to Examples described below.

### (Example 1)

### [First step]

To a stainless steel reaction vessel with a capacity of 40 mL, PdCl₂ (palladium chloride; manufactured by Soekawa Chemical Co., Ltd.; 3.0 mg, 17.01 µmol) as the compound having at least one element selected from Periodic Table Group 10 elements, which is the first catalyst, triphenylphosphine (manufactured by Tokyo Chemical Industry Co., Ltd.; 8.92 mg, 34.00 µmol) as the organic phosphine ligand which is the first catalyst, and PTSA (p-toluenesulfonic acid. monohydrate; manufactured by Tokyo Chemical Industry Co., Ltd.; 1.3 mg, 6.723 µmol) as the acidic compound which is the first catalyst were added.

To the reaction vessel in which these first catalysts are charged, vinyl acetate (manufactured by FUJIFILM Wako Pure Chemical Corporation; 0.75 mL, 8.1 mmol), ethanol (manufactured by FUJIFILM Wako Pure Chemical Corporation; 1.2 mL, 20 mmol) as the primary or secondary alcohol, toluene (manufactured by FUJIFILM Wako Pure Chemical Corporation; 6.75 mL) as the solvent, and pyridine (manufactured by FUJIFILM Wako Pure Chemical Corporation; 0.0275 mL, 340.2 µmol) which is the nitrogen atom-containing heterocyclic compound as the first catalyst were added under a nitrogen stream to obtain a reaction solution.

A used amount of the palladium chloride in Example 1 was 0.002 molar part with respect to 1 molar part of the vinyl acetate. A used amount of the organic phosphine ligand was 2 molar parts with respect to 1 molar part of the palladium chloride. A used amount of PTSA (p-toluenesulfonic acid. monohydrate) was 0.395 molar parts of acid point of PTSA with respect to 1 molar part of the palladium chloride. A used amount of the pyridine was 20 molar parts with respect to 1 molar part of the palladium chloride.

In addition, a used amount of the toluene was 0.83 liters (amount in which the concentration of the vinyl acetate in the reaction solution was 1.2 mol/liter) with respect to 1 molar part of the ethanol.

Next, using a carbon monoxide gas, pressurization and depressurization in the reaction vessel was repeated 3 times between atmospheric pressure and 1 MPa, and the gas inside the reaction vessel was replaced with the carbon monoxide gas. Thereafter, the pressure inside the reaction vessel was increased to 5 MPa using the carbon monoxide gas, and the reaction solution was reacted at 100°C for 15 hours while stirring to produce a first ester compound.

The reaction solution after the reaction was analyzed using gas chromatography (device name: 6850GC, manufactured by Agilent Technologies, Inc.). Quantitative analysis of the raw material, the first ester compound which is the target product, and the side reaction product was performed by creating a calibration curve using diethylene glycol dimethyl ether (manufactured by Tokyo Chemical Industry Co., Ltd.) as an internal standard substance.

As a result, in the first step of Example 1, it was confirmed that the first ester compound in which R¹ in General Formula (2) was an ethyl group was produced. A conversion rate of the vinyl acetate was 29%, the yield of the first ester compound based on the vinyl acetate used in the first step was 26%, and the yield of the ethyl acetate based on the vinyl acetate used in the first step was 3%.

### [Intermediate step]

10 mg of a catalyst (manufactured by N.E. CHEMCAT CORPORATION) in which palladium was supported on a carrier consisting of activated carbon by 5 parts by mass with respect to 100 parts by mass of the carrier was added to the reaction solution after the first step as a catalyst. Thereafter, using a nitrogen gas, pressurization and depressurization in the reaction vessel was repeated 3 times between atmospheric pressure and 1 MPa, and the gas inside the reaction vessel was replaced with the nitrogen gas. Thereafter, the inside of the reaction vessel was set to a hydrogen gas atmosphere of 0.8 MPa, and the reaction was carried out at room temperature (20°C) for 15 hours to reduce unreacted vinyl acetate included in the reaction solution.

In the same manner as in the reaction solution after the first step, the reaction solution after the reaction was analyzed using gas chromatography (device name: 6850GC, manufactured by Agilent Technologies, Inc.).

As a result, it was confirmed that the vinyl acetate disappeared and the ethyl acetate was produced by performing the intermediate step in Example 1. The yield of the first ester compound after the intermediate step based on the vinyl acetate used in the first step was 26%, and the yield of the ethyl acetate based on the vinyl acetate used in the first step was 71%.

### [Second step]

Using an evaporator, low boiling point compounds (boiling point of 120°C or lower) such as ethanol, ethyl acetate, and toluene were recovered and removed from the reaction solution after the intermediate step. From the reaction solution after the intermediate step, in which the low boiling point compounds had been removed, the first ester compound was isolated using a silica column (manufactured by FUJIFILM Wako Pure Chemical Corporation) using ethyl acetate as an eluent.

The isolated first ester compound (41.6 mg, 0.26 mmol), ethanol dried in advance with Molecular Sieve 3A (manufactured by UNION SHOWA K.K.) (3 ml, 0.051 mol), and potassium carbonate (400 mg, 2.9 mmol) as a catalyst were added to an eggplant-shaped flask, and the reaction was carried out by stirring the mixture at room temperature (20°C) and normal pressure for 1 hour.

In the same manner as in the reaction solution after the first step, the reaction solution after the reaction was analyzed using gas chromatography (device name: 6850GC, manufactured by Agilent Technologies, Inc.). Quantitative analysis of the lactic acid ester represented by General Formula (3) and the acetic acid ester represented by General Formula (4), which are the target products, and the side reaction product was performed by creating a calibration curve using diethylene glycol dimethyl ether (manufactured by Tokyo Chemical Industry Co., Ltd.) as an internal standard substance.

As a result, it was confirmed that, by performing the second step of Example 1, the lactic acid ester represented by General Formula (3), in which R¹ was an ethyl group, and the acetic acid ester represented by General Formula (4), in which R¹ was an ethyl group, were produced. The yield of the lactic acid ester based on the first ester compound used in the second step was 63%, and the yield of the acetic acid ester based on the first ester compound used in the second step was 62%.

### (Example 2)

### [First step]

The first step was performed in the same manner as in Example 1, except that 2-dicyclohexylphosphino-2',6'-dimethoxybiphenyl represented by Formula (5-1) (SPhos; manufactured by Sigma-Aldrich Japan; 14.0 mg, 0.034 mmol) was used as the organic phosphine ligand which is the first catalyst, and methanol (manufactured by FUJIFILM Wako Pure Chemical Corporation; 0.81 mL; 20 mmol) was used as the primary or secondary alcohol.

Same as Example 1, the reaction solution after the reaction was analyzed using gas chromatography (device name: 6850GC, manufactured by Agilent Technologies, Inc.).

As a result, in the first step of Example 2, it was confirmed that the first ester compound in which R¹ in General Formula (2) was a methyl group was produced. A conversion rate of the vinyl acetate was 95%, the yield of the first ester compound based on the vinyl acetate used in the first step was 79%, and the yield of the methyl acetate based on the vinyl acetate used in the first step was 9%.

### [Second step]

Using an evaporator, low boiling point compounds (boiling point of 120°C or lower) such as methanol, methyl acetate, and toluene were recovered and removed from the reaction solution after the first step. From the reaction solution after the first step, in which the low boiling point compounds had been removed, the first ester compound was isolated using a silica column (manufactured by FUJIFILM Wako Pure Chemical Corporation) using ethyl acetate as an eluent.

The isolated first ester compound (43.8 mg, 0.30 mmol), methanol dried in advance with Molecular Sieve 3A (manufactured by UNION SHOWA K.K.) (3 ml, 0.074 mol), and potassium carbonate (400 mg, 2.9 mmol) as a catalyst were added to an eggplant-shaped flask, and the reaction was carried out by stirring the mixture at room temperature (20°C) and normal pressure for 1 hour.

In the same manner as in the reaction solution after the second step of Example 1, the reaction solution after the reaction was analyzed using gas chromatography (device name: 6850GC, manufactured by Agilent Technologies, Inc.).

As a result, it was confirmed that, by performing the second step of Example 2, the lactic acid ester represented by General Formula (3), in which R¹ was a methyl group, and the acetic acid ester represented by General Formula (4), in which R¹ was a methyl group, were produced. The yield of the lactic acid ester based on the first ester compound used in the second step was 75%, and the yield of the acetic acid ester based on the first ester compound used in the second step was 76%.

### (Example 3)

### [First step]

The first step was performed in the same manner as in Example 1, except that 2-dicyclohexylphosphino-2',6'-dimethoxybiphenyl represented by Formula (5-1) (SPhos; manufactured by Sigma-Aldrich Japan; 14.0 mg, 0.034 mmol) was used as the organic phosphine ligand which is the first catalyst.

Same as Example 1, the reaction solution after the reaction was analyzed using gas chromatography (device name: 6850GC, manufactured by Agilent Technologies, Inc.).

As a result, in the first step of Example 3, it was confirmed that the first ester compound in which R¹ in General Formula (2) was an ethyl group was produced. A conversion rate of the vinyl acetate was 96%, the yield of the first ester compound based on the vinyl acetate used in the first step was 85%, and the yield of the ethyl acetate based on the vinyl acetate used in the first step was 7%.

### [Intermediate step]

50 mg of a catalyst (manufactured by N.E. CHEMCAT CORPORATION) in which palladium was supported on a carrier consisting of activated carbon by 10 parts by mass with respect to 100 parts by mass of the carrier was added to the reaction solution after the first step as a catalyst, and the intermediate step was performed in the same manner as in Example 1.

In the same manner as in the reaction solution after the first step, the reaction solution after the reaction was analyzed using gas chromatography (device name: 6850GC, manufactured by Agilent Technologies, Inc.).

As a result, it was confirmed that the vinyl acetate disappeared and the ethyl acetate was produced by performing the intermediate step in Example 3. The yield of the first ester compound after the intermediate step based on the vinyl acetate used in the first step was 84%, and the yield of the ethyl acetate based on the vinyl acetate used in the first step was 12%.

### [Second step]

The reaction solution after the intermediate step (7.0 g, containing 1090 mg of the first ester compound), ethanol dried in advance with Molecular Sieve 3A (manufactured by UNION SHOWA K.K.) (2.0 ml, 34 mmol), and potassium carbonate (1000 mg, 7.2 mmol) as a catalyst were added to an eggplant-shaped flask, and the reaction was carried out by stirring the mixture at room temperature (20°C) and normal pressure for 1 hour.

In the same manner as in the reaction solution after the second step of Example 1, the reaction solution after the reaction was analyzed using gas chromatography (device name: 6850GC, manufactured by Agilent Technologies, Inc.).

As a result, it was confirmed that, by performing the second step of Example 3, the lactic acid ester represented by General Formula (3), in which R¹ was an ethyl group, and the acetic acid ester represented by General Formula (4), in which R¹ was an ethyl group, were produced. The yield of the lactic acid ester based on the first ester compound used in the second step was 86%, and the yield of the acetic acid ester based on the vinyl acetate used in the first step was 98%.

## Claims

1. A method for producing an ester compound, comprising:
a first step of reacting a vinyl acetate with a primary or secondary alcohol represented by General Formula (1) and carbon monoxide in a presence of a first catalyst to produce a first ester compound represented by General Formula (2); and
a second step of reacting the first ester compound with an alcohol in a presence of a second catalyst to produce a lactic acid ester represented by General Formula (3) and an acetic acid ester represented by General Formula (4),
R¹OH ... (1) 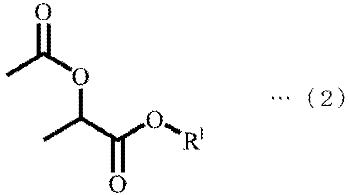 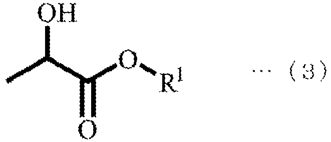 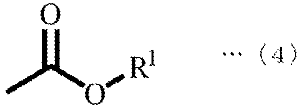
(in Formulae (1) to (4), R¹ is a hydrocarbon group which has 1 to 10 carbon atoms and may have an alicyclic ring or an aromatic ring in part or in whole, R¹'s in Formula (1) and Formula (2) are the same, and R¹'s in Formula (3) and Formula (4) are the same).

2. The method for producing an ester compound according to Claim 1, further comprising, before the second step:
an intermediate step of reducing an unreacted vinyl acetate included in a reaction solution after the first step to convert the unreacted vinyl acetate into an acetic acid ester.

3. The method for producing an ester compound according to Claim 2,
wherein, in the intermediate step, an acetaldehyde included in the reaction solution after the first step is hydrogenated to be converted into ethanol, and
in the second step, the first ester compound in the reaction solution after the intermediate step is reacted with the ethanol in the presence of the second catalyst.

4. The method for producing an ester compound according to any one of Claims 1 to 3,
wherein the primary or secondary alcohol represented by General Formula (1) is ethanol.

5. The method for producing an ester compound according to any one of Claims 1 to 4,
wherein the first catalyst includes a compound having at least one element selected from Periodic Table Group 10 elements, and an organic phosphine ligand.

6. The method for producing an ester compound according to Claim 5,
wherein the Periodic Table Group 10 element is Pd.

7. The method for producing an ester compound according to Claim 5,
wherein the organic phosphine ligand is a monodentate organic phosphine ligand.

8. The method for producing an ester compound according to Claim 7,
wherein the organic phosphine ligand is an organic phosphine ligand represented by General Formula (5), 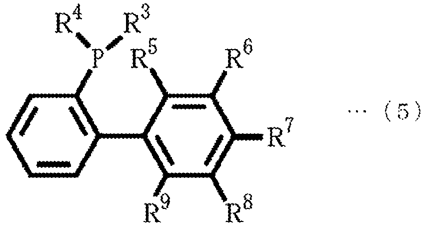
(in Formula (5), R³ and R⁴ are alkyl groups having 1 to 6 carbon atoms, cycloalkyl groups having 6 to 9 carbon atoms, or aryl groups having 6 to 9 carbon atoms, R³ and R⁴ may be the same or different from each other, R⁵ to R⁹ are hydrogen atoms, alkyl groups having 1 to 4 carbon atoms, alkoxy groups having 1 to 4 carbon atoms, or dialkylamino groups having 2 to 8 carbon atoms, and R⁵ to R⁹ may all be the same or may be partially or completely different from each other).

9. The method for producing an ester compound according to any one of Claims 1 to 8,
wherein the first catalyst includes an acidic compound.

10. The method for producing an ester compound according to Claim 9,
wherein the acidic compound is at least one selected from the group consisting of an alkyl sulfonic acid, an ammonium salt of an alkyl sulfonic acid, a pyridinium salt of an alkyl sulfonic acid, an aryl sulfonic acid, an ammonium salt of an aryl sulfonic acid, and a pyridinium salt of an aryl sulfonic acid.

11. The method for producing an ester compound according to any one of Claims 1 to 10,
wherein the first catalyst includes a nitrogen atom-containing heterocyclic compound.

12. The method for producing an ester compound according to Claim 11,
wherein the nitrogen atom-containing heterocyclic compound is at least one selected from the group consisting of pyridine and picoline.

13. The method for producing an ester compound according to any one of Claims 1 to 12,
wherein the second catalyst is at least one selected from the group consisting of potassium carbonate, sodium methoxide, sodium ethoxide, sodium-t-butoxide, potassium ethoxide, tetraethoxytitanium, tetraisopropoxytitanium, tetra-n-butoxytitanium, mono-n-butyltin oxide, di-n-butyltin oxide, di-n-butyltin laurate, and di-n-octyltin oxide.

14. The method for producing an ester compound according to any one of Claims 1 to 13, further comprising:
before the first step, a carbon monoxide producing step of pyrolyzing a plastic or a biomaterial to produce carbon monoxide.

15. The method for producing an ester compound according to Claim 4,
wherein the ethanol is bioethanol derived from a plant.

16. A method for producing an ester compound, comprising:
a first step of reacting a vinyl acetate with a primary or secondary alcohol represented by General Formula (1) and carbon monoxide in a presence of a first catalyst to produce a first ester compound represented by General Formula (2),
wherein the first catalyst includes a compound having at least one element selected from Periodic Table Group 10 elements, and an organic phosphine ligand represented by General Formula (5),
R¹OH ... (1) 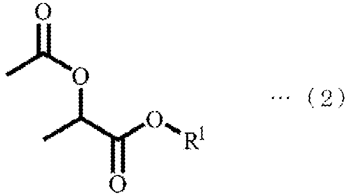 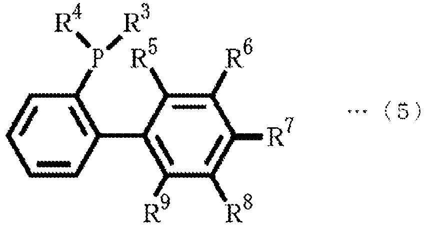
(in Formulae (1) and (2), R¹ is a hydrocarbon group which has 1 to 10 carbon atoms and may have an alicyclic ring or an aromatic ring in part or in whole, and R¹'s in Formula (1) and Formula (2) are the same)
(in Formula (5), R³ and R⁴ are alkyl groups having 1 to 6 carbon atoms, cycloalkyl groups having 6 to 9 carbon atoms, or aryl groups having 6 to 9 carbon atoms, R³ and R⁴ may be the same or different from each other, R⁵ to R⁹ are hydrogen atoms, alkyl groups having 1 to 4 carbon atoms, alkoxy groups having 1 to 4 carbon atoms, or dialkylamino groups having 2 to 8 carbon atoms, and R⁵ to R⁹ may all be the same or may be partially or completely different from each other).

17. The method for producing an ester compound according to Claim 16,
wherein the primary or secondary alcohol represented by General Formula (1) is ethanol.

18. The method for producing an ester compound according to Claim 16 or 17,
wherein the Periodic Table Group 10 element is Pd.

19. The method for producing an ester compound according to any one of Claims 16 to 18,
wherein, in General Formula (5), R³ and R⁴ are cyclohexyl groups, R⁵ and R⁹ are methoxy groups, and R⁶ to R⁸ are hydrogen atoms.

20. The method for producing an ester compound according to any one of Claims 16 to 19,
wherein the first catalyst includes an acidic compound.

21. The method for producing an ester compound according to Claim 20,
wherein the acidic compound is at least one selected from the group consisting of an alkyl sulfonic acid, an ammonium salt of an alkyl sulfonic acid, a pyridinium salt of an alkyl sulfonic acid, an aryl sulfonic acid, an ammonium salt of an aryl sulfonic acid, and a pyridinium salt of an aryl sulfonic acid.

22. The method for producing an ester compound according to any one of Claims 16 to 21,
wherein the first catalyst includes a nitrogen atom-containing heterocyclic compound.

23. The method for producing an ester compound according to Claim 22,
wherein the nitrogen atom-containing heterocyclic compound is at least one selected from the group consisting of pyridine and picoline.
